# DEMANDE DE BREVET EUROPEEN

(11) **EP 0 903 129 A1**
(43) Date de publication de la demande: **24.03.1999**
(21) Numéro de dépôt: 98440192.7
(22) Date de dépôt: 02.09.1998
(51) Int. Cl.: A61F 5/058

(54) **Dispositif de contention destine plus particulierement au maintien d'une partie du corps humain**

(30) Priorité: 15.09.1997 FR 9711614
(71) Demandeur: Ragot Claude Sàrl, 69400 Arnas (FR); Dumont-Securite S.A., 01500 Amberieu en Bugey (FR)
(72) Inventeur: Ragot, Claude, 01480 Jassans Riottier (FR)
(74) Mandataire: Arbousse-Bastide, Jean-Claude Philippe

(57) **Abrégé**

Dispositif de contention destiné plus particulièrement au maintien d'une partie du corps humain, du type comprenant un matelas constitué d'une enveloppe externe étanche (1) renfermant des billes (2) de matériau semi-rigide et munie d'une valve (12) pour sa mise en dépression.

Il comprend en outre une enveloppe interne non étanche (3,3') qui entoure les billes (2) à l'intérieur de l'enveloppe externe (1) et dont la structure est réticulée de manière à immobiliser les billes (2) lors de la mise en dépression de l'enveloppe externe (1).

## Description

La présente invention a pour objet un dispositif de contention destiné plus particulièrement au maintien d'une partie du corps humain.

Actuellement les dispositifs de contention du type à immobilisation sont constitués d'une enveloppe étanche à l'intérieur de laquelle sont disposés des billes de matériau semi-déformable, notamment de polystyrène, et qui est destinée à être enroulée autour d'une partie d'un corps humain accidenté en vue de le maintenir dans une position voulue en créant une dépression dans ladite enveloppe pour provoquer une agglomération des billes et former un matelas rigide immobilisant ladite partie du corps humain.

Toutefois ces dispositifs présentent plusieurs directions de rétraction, ce qui peut avoir des effets dommageables, notamment en cas de lésion de la colonne vertébrale. De plus l'enveloppe au cours de la mise en dépression vient s'intercaler partiellement entre les billes, rendant la surface du dispositif irrégulière.

Pour pallier cet inconvénient le document FR-A-2579088 décrit un dispositif à réduction dimensionnelle actionné par dépression, constitué par au moins une enveloppe étanche munie d'une buse de raccordement à un organe déprimogène et renfermant un corps souple rétractable formé d'éléments en matière souple à propriétés d'écrasement différentes et présentant au moins une direction de rétraction préférentielle, une dépression dans ladite enveloppe ayant pour effet un fronçage de ses parois perpendiculairement à la direction de rétraction, les éléments de moindre écrasement formant des raidisseurs longitudinaux.

Cependant ce dispositif est de réalisation complexe et s'il diminue les effets de rétraction dans le sens longitudinal, le résultat demeure insuffisant. D'autre part il présente des problèmes de solidité du fait des éléments raidisseurs qui, à la longue, peuvent détériorer l'enveloppe étanche en la déchirant. En outre les éléments raidisseurs forment des écrans qui peuvent affecter la clarté des radiographies du patient.

La présente invention a pour but de remédier à ces inconvénients en proposant un dispositif de contention qui permet de limiter fortement les effets de rétraction dans n'importe quelle direction tout en conservant à l'enveloppe une surface parfaitement lisse.

Le dispositif de contention selon la présente invention est du type comprenant un matelas constitué d'une enveloppe externe étanche renfermant des billes de matériau semi-rigide et munie d'une valve pour sa mise en dépression, et il se caractérise essentiellement en ce qu'il comprend en outre une enveloppe interne non étanche qui entoure les billes à l'intérieur de l'enveloppe externe et dont la structure est réticulée de manière à immobiliser les billes lors de la mise en dépression du dispositif.

L'enveloppe interne peut avantageusement être réalisée en une matière tissée, en un matériau moulé en forme de tissage, tel qu'un polymère du type polyester, ou en une feuille perforée.

Les dimensions relatives des billes et des mailles de l'enveloppe interne doivent être telles que les mailles puissent retenir les billes de manière à les empêcher de se regrouper de manière désordonnée lors de la mise en dépression du dispositif.

L'enveloppe interne du dispositif selon l'invention présentera avantageusement une surface de mailles qui n'excèdera pas 0,4 mm², tandis que les billes présenteront de préférence un diamètre supérieur ou égal à 0,2 mm.

Dans un mode de réalisation préférentiel de l'invention l'enveloppe étanche et l'enveloppe interne sont constituées chacune de deux parties solidarisées bord à bord avec les deux autres parties.

Les avantages et les caractéristiques de la présente invention ressortiront plus clairement de la description qui suit et qui se rapporte au dessin annexé, étant entendu que cette description ne présente aucun caractère limitatif vis à vis de l'invention.

Dans le dessin annexé :
- la figure 1 représente une vue en perspective, avec arraché partiel, du dispositif de contention selon l'invention dans un mode de réalisation préférentiel.
- la figure 2 représente une vue en coupe du même dispositif.

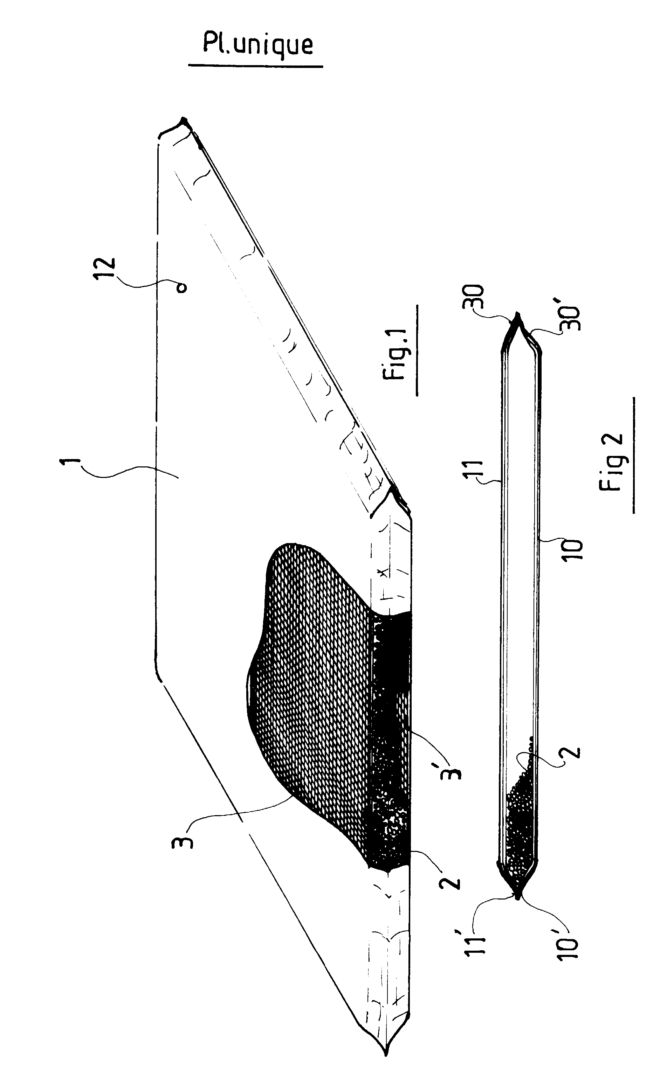

Si on se réfère à la figure 1 on peut voir que le dispositif de contention selon l'invention présente la forme d'un matelas constitué d'une enveloppe externe 1 étanche de tissu enduit, par exemple de polyuréthane ou de PVC, munie d'une valve 12 pour la mise en dépression de son espace intérieur.

L'enveloppe 1 renferme un certain volume de billes 2 en polystyrène, et deux toiles tissées 3 et 3' en polyester disposée entre les billes 2 qu'elles entourent et l'enveloppe externe 1.

L'enveloppe étanche 1 est réalisée en deux parties 10 et 11 solidarisées par collage ou soudage de leurs bords 10',11' avec les bords 30,30' des deux toiles 3 et 3', comme on peut le voir également sur la figure 2.

Le matelas selon l'invention comporte également une structure portante comprenant des poignées, non représentée.

Le fonctionnement du dispositif selon l'invention est le suivant:

On place dans un premier temps le matelas autour du corps humain accidenté ou autour d'une partie de celui-ci, puis on crée une dépression dans l'enveloppe étanche 1, dont les billes 2 sont bloquées en position du fait de la structure maillée des toiles 3 et 3' en contact avec elles, ce qui a pour effet de former une coque rigide avec une très faible rétraction de l'ensemble dans n'importe quelle direction. En outre les toiles 3 et 3', en retenant les billes, empêchent l'enveloppe externe de se déformer en surface sous l'effet de la dépression.

## Revendications

1. Dispositif de contention destiné plus particulièrement au maintien d'une partie du corps humain, du type comprenant un matelas constitué d'une enveloppe externe étanche (1) renfermant des billes (2) de matériau semi-rigide et munie d'une valve (12) pour sa mise en dépression, caractérisé en ce qu'il comprend en outre une enveloppe interne non étanche (3,3') qui entoure les billes (2) à l'intérieur de ladite enveloppe externe (1) et dont la structure est réticulée de manière à immobiliser les billes (2) lors de la mise en dépression de l'enveloppe externe (1).

2. Dispositif selon la revendication 1 caractérisé en ce que l'enveloppe interne (3,3') est réalisée en une matière tissée en un matériau moulé en forme de tissage ou en une feuille perforée.

3. Dispositif selon la revendication 2 caractérisé en ce que la matière tissée ou le matériau moulé est un polymère du type polyester.

4. Dispositif selon la revendication 2 ou 3 caractérisé en ce que l'enveloppe externe étanche (1) et l'enveloppe interne (2) sont constituées chacune de deux parties solidarisées bord à bord avec les deux autres parties.
